(19)

**Europäisches Patentamt**
**European Patent Office**
**Office européen des brevets**

(11) **EP 4 678 104 A1**

(12) **EUROPEAN PATENT APPLICATION**

(43) Date of publication:
**14.01.2026 Bulletin 2026/03**

(21) Application number: **25184341.3**

(22) Date of filing: **21.06.2025**

(51) International Patent Classification (IPC):
***A61B 5/361*** (2021.01)      ***A61B 5/00*** (2006.01)

(52) Cooperative Patent Classification (CPC):
**A61B 5/361; A61B 5/7267;** A61B 5/352

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.07.2024 IN 202421052050**

(71) Applicant: **Tata Consultancy Services Limited**
**Mumbai, Maharashtra 400 021 (IN)**

(72) Inventors:
 • **DEB, Trisrota**
  **700160 Kolkata, West Bengal (IN)**
 • **SAHU, Ishan**
  **700160 Kolkata, West Bengal (IN)**

 • **UKIL, Arijit**
  **700160 Kolkata, West Bengal (IN)**
 • **PAL, Arpan**
  **700160 Kolkata, West Bengal (IN)**
 • **GARAIN, Utpal**
  **700108 Kolkata, West Bengal (IN)**
 • **SAHA, Saptarshi**
  **700160 Kolkata, West Bengal (IN)**
 • **SAHOO, Pratyush Kumar**
  **700160 Kolkata, West Bengal (IN)**
 • **MANDANA, Kayapanda Muthana**
  **700107 Kolkata, West Bengal (IN)**

(74) Representative: **Goddar, Heinz J.**
 **Boehmert & Boehmert**
 **Anwaltspartnerschaft mbB**
 **Pettenkoferstrasse 22**
 **80336 München (DE)**

(54) **METHOD AND SYSTEM FOR EXPLAINING DECISION-MAKING PROCESS OF MODEL DETECTING ATRIAL FIBRILLATION IN ELECTROCARDIOGRAM WAVES**

(57)      Current approaches for atrial fibrillation (AF) detection use deep learning models which remain opaque. **In** particular, they lack in providing explanation of why this particular decision (around existence of AF) has been made, thereby making it unacceptable to clinical domain experts. Present disclosure provides method and system for explaining decision-making process of deep learning models used for detecting AF in ECG waves. The system receives ECG signal which is converted into two-dimensional (2D) representation which further helps in classification of diagnosis condition from ECG signal using classifier model. Thereafter, system generates class activation maps (CAM) to find attention scores and finally uses these attention scores, to identify top **R-R** intervals where classifier model is placing greater emphasis. Further, system converts ECG image into ECG signal which also converts CAM into attention wave. Finally, system uses ECG signal and attention wave to generate clinical expert like explanations for class label prediction.

FIG. 4

**Description**

CROSS-REFERENCE TO RELATED APPLICATIONS AND PRIORITY

The present application claims priority to Indian application no. 202421052050, filed on July 8, 2024.

TECHNICAL FIELD

The disclosure herein generally relates to atrial fibrillation detection in electrocardiogram (ECG) signal, and, more particularly, to a method and a system for explaining decision-making process of deep learning models used for detecting atrial fibrillation in ECG waves.

BACKGROUND

Atrial fibrillation (AF) stands as the most commonly encountered arrhythmia, associated with higher mortality rates and increased risks of ischemic stroke, heart failure, and dementia among patients. AF is acknowledged as a 21st century cardiovascular disease epidemic. Electrical recordings of cardiac activity, like the 12-lead electrocardiogram (ECG), offer valuable insights into cardiovascular well-being of a person. In general, ECGs serve as a primary diagnostic tool for identifying AF in clinical practice.

Recently, deep learning models have demonstrated promising results in autonomously identifying the existence of atrial fibrillation in ECG. However, in practical clinical scenarios, achieving accurate classification is paramount, but equally vital is the interpretability of results. Further, certain heart conditions may not consistently manifest abnormal ECG patterns, particularly in the early stages of the disease. Hence, ensuring the interpretability of results, especially in spotlighting diagnosis-relevant aspects of the data, becomes imperative for early detection and informed clinical decision-making.

As deep learning models remain opaque 'black boxes', they lack in providing the thorough interpretability of results necessary for practical clinical applications. In particular, they lack in providing explanation of why this particular decision (around existence of atrial fibrillation) has been made, thereby making it unacceptable to clinical domain experts.

SUMMARY

Embodiments of the present disclosure present technological improvements as solutions to one or more of the above-mentioned technical problems recognized by the inventors in conventional systems. For example, in one aspect, there is provided a processor implemented method for explaining decision-making process of deep learning models used for detecting atrial fibrillation in electrocardiogram (ECG) waves. The method comprises receiving, by a system via one or more hardware processors, a one-dimensional (1D) ECG signal; converting, by the system via the one or more hardware processors, the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image; training, by the system via the one or more hardware processors, a deep learning classifier model based on the 2D ECG image and a clinical domain knowledge, to obtain a trained deep learning classifier model, wherein the trained deep learning classifier model provides a class label among one or more predefined class labels, for the 2D ECG image, and wherein the one or more predefined class labels comprises an Atrial Fibrillation (AF) rhythm label and a normal sinus rhythm label; applying, by the system via the one or more hardware processors, an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels; converting, by the system via the one or more hardware processors, the 2D ECG image into the 1D ECG signal using a 2D-1D image to signal conversion algorithm, wherein the 2D-1D image to signal conversion algorithm further converts the 2D CAM into a 1D attention wave; calculating, by the system via the one or more hardware processors, a plurality of attention scores from a plurality of R-R intervals present in the 1D ECG signal based on the 1D attention wave; and sorting, by the system via the one or more hardware processors, the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

In an embodiment, the 2D-1D image to signal conversion algorithm converts the 2D ECG image into the 1D ECG signal by performing: scanning each segment of the plurality of segments present in the 2D ECG image; discarding each segment amongst the plurality of segments that is zero-padded to obtain a subset of image segments; and concatenating each segment of the subset of image segments from a first segment to a last segment of the subset of image segments to generate the 1D ECG signal.

In an embodiment, the 2D-1D image to signal conversion algorithm converts the 2D CAM into the 1D attention wave by performing: scanning each each CAM segment of a plurality of CAM segments present in the 2D CAM; discarding each CAM segment amongst the plurality of CAM segments that is zero-padded to obtain a subset of CAM segments; and concatenating each CAM segment of the subset of CAM segments from a first CAM segment to a last CAM segment of the subset of CAM segments to generate the 1D attention wave.

In an embodiment, the plurality of attention scores are calculated by performing: scanning, by the system via the one or more hardware processors, each R-R interval of the plurality of R-R intervals present in the 1D ECG signal to obtain a length corresponding to each R-R interval; computing, by the system via the one or more hardware processors, an area corresponding to the 1D attention wave under each R-R interval using a Trapezoidal Rule; and normalizing, by the system via the one or more hardware processors, the area for each R-R interval by dividing the area with the length of each R-R interval to obtain an attention score, wherein attention scores obtained corresponding to the plurality of R-R intervals form the plurality of attention scores.

In another aspect, there is provided a system for explaining decision-making process of deep learning models used for detecting atrial fibrillation in electrocardiogram (ECG) waves. The system comprises a memory storing instructions; one or more communication interfaces; and one or more hardware processors coupled to the memory via the one or more communication interfaces, wherein the one or more hardware processors are configured by the instructions to: receive a one-dimensional (1D) electrocardiogram (ECG) signal; convert the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image; train a deep learning classifier model based on the 2D ECG image and a clinical domain knowledge, to obtain a trained deep learning classifier model, wherein the trained deep learning classifier model provides a class label among one or more predefined class labels for the 2D ECG image, wherein the one or more predefined class labels comprises an Atrial Fibrillation (AF) rhythm label and a normal sinus rhythm label; apply an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels; convert the 2D ECG image into the 1D ECG signal using a 2D-1D image to signal conversion algorithm, wherein the 2D-1D image to signal conversion algorithm further converts the 2D CAM into a 1D attention wave; calculate a plurality of attention scores from a plurality of R-R intervals present in the 1D ECG signal based on the 1D attention wave; and sort the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

In an embodiment, for converting the 2D ECG image into the 1D ECG signal using the 2D-1D image to signal conversion algorithm, the one or more hardware processors (204) are configured by the instructions to: scan each segment of the plurality of segments present in the 2D ECG image; discard each segment amongst the plurality of segments that is zero-padded to obtain a subset of image segments; and concatenate each segment of the subset of image segments from a first segment to a last segment of the subset of image segments to generate the 1D ECG signal.

In an embodiment, for converting the 2D CAM into the 1D attention wave using the 2D-1D image to signal conversion algorithm, the one or more hardware processors (204) are configured by the instructions to: scan each CAM segment of a plurality of CAM segments present in the 2D CAM; discard each CAM segment amongst the plurality of CAM segments that is zero-padded to obtain a subset of CAM segments; and concatenate each CAM segment of the subset of CAM segments from a first CAM segment to a last CAM segment of the subset of CAM segments to generate the 1D attention wave.

In an embodiment, for calculating the plurality of attention scores, the one or more hardware processors (204) are configured by the instructions to: scan each R-R interval of the plurality of R-R intervals present in the 1D ECG signal to obtain a length corresponding to each R-R interval; compute an area corresponding to the 1D attention wave under each R-R interval using a Trapezoidal Rule; and normalize the area for each R-R interval by dividing the area with length of each R-R interval to obtain an attention score, wherein attention scores obtained corresponding to the plurality of R-R intervals form the plurality of attention scores.

In yet another aspect, there are provided one or more non-transitory machine-readable information storage mediums comprising one or more instructions which when executed by one or more hardware processors cause a method for explaining decision-making process of deep learning models used for detecting atrial fibrillation in electrocardiogram (ECG) waves. The method comprises receiving, by a system, a one-dimensional (1D) ECG signal; converting, by the system, the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image; training, by the system, a deep learning classifier model based on the 2D ECG image and a clinical domain knowledge, to obtain a trained deep learning classifier model, wherein the trained deep learning classifier model provides a class label among one or more predefined class labels, for the 2D ECG image, wherein the one or more predefined class labels comprises an Atrial Fibrillation (AF) rhythm label and a normal sinus rhythm label; applying, by the system, an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels; converting, by the system, the 2D ECG image into the 1D ECG signal using a 2D-1D image to signal conversion algorithm, wherein the 2D-1D image to signal conversion algorithm further converts the 2D CAM into a 1D attention wave; calculating, by the system, a plurality of attention scores from a plurality of R-R intervals present in the 1D ECG signal based on the 1D attention wave; and sorting, by the system, the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a schematic representation of an electrocardiogram (ECG) waveform, in accordance with some embodiments of the present disclosure.
FIG. 2 illustrates an exemplary representation of an environment 200 related to at least some example embodiments of the present disclosure.
FIG. 3 illustrates an exemplary block diagram of a system for explaining decision-making process of deep learning models used for detecting atrial fibrillation in ECG waves, in accordance with an embodiment of the present disclosure.
FIG. 4 is a schematic flow diagram representation illustrating working of the system of FIGS. 2 and 3, in accordance with an embodiment of the present disclosure.
FIGS. 5A and 5B collectively referred to as FIG. 5 illustrates an exemplary flow diagram of a method for explaining decision-making process of deep learning models used for detecting atrial fibrillation in ECG waves using the system of FIGS. 2 and 3, in accordance with an embodiment of the present disclosure.
FIG. 6 is an example representation of a two-dimensional ECG image obtained from a one-dimensional ECG signal, in accordance with an embodiment of the present disclosure.
FIG. 7 is an example representation of the one-dimensional ECG signal obtained from the two-dimensional ECG image, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As discussed earlier, deep learning systems can outperform conventional algorithms in performing automated identification of atrial fibrillation (AF). However, understanding how deep learning algorithms/systems make their decisions is notoriously hard, particularly in the context of electrocardiogram (ECG) classification as due to the black box nature of the deep learning algorithms/systems, they do not provide the explanation on why this particular decision has been made. This further acts as a barrier in widespread clinical adoption of deep learning systems for detecting AF in ECG signals.

So, a technique that can provide purpose of detecting atrial fibrillation and can explain decision-making process followed by the deep learning model for detecting AF while being accepted by clinical domain experts is still to be explored.

Embodiments of the present disclosure overcome the above-mentioned disadvantages by providing a method and a system for explaining decision-making process of deep learning models used for detecting atrial fibrillation in ECG waves. In particular, the system is an artificial intelligence (AI) based assistive solution that can increase efficiency and confidence of doctors who are not specialized in cardiology (for e.g., primary care doctors, gynecologists) but need to take clinical decisions from ECG recordings by providing them information associated with a purpose of classification of ECG signals and explaining the decision-making process followed by a deep learning based model to come up with the classification/analysis (i.e., detection of AF).

The system of the present disclosure first receives one-dimensional (1D) electrocardiogram (ECG) signal from a data source/user. The system then converts the 1D ECG signal to a two-dimensional (2D) representation which helps in the classification of a diagnosis condition from the ECG signal using a deep learning classifier model. In particular, the 2D representation capture relative heartbeat information concurrently which is further utilized by the in classifying the disease diagnosis condition from the ECG signal. Thereafter, the system generates class activation maps (CAM) to find attention scores and finally uses these attention scores, to identify top R-R intervals where the deep learning classifier model is placing greater emphasis to make its decision. Further, the system converts the 2D ECG image into the 1D ECG signal using a 2D-1D image to signal conversion algorithm which also converts the CAM into a 1D attention wave. Finally, the system uses the1D ECG signal and the 1D attention wave to generate clinical expert like explanations for the class label prediction performed by the deep learning classifier model.

In the present disclosure, the system converts the class activation maps into a 1D attention wave which along with the clinical domain knowledge helps in investigating specific region of interests (ROIs) in the input ECG. The ROIs further helps in gaining insight into whether the deep learning classifier model is prioritizing irregularities in the R-R intervals and the absence of P waves to come up with classification or not, thus providing clinical expert like explanations for the classification decision while ensuring increased accuracy of the AF detection. Further, the system approach is closely aligned with how cardiologists analyze an ECG signal, thereby making the diagnosis more acceptable for clinical domain experts.

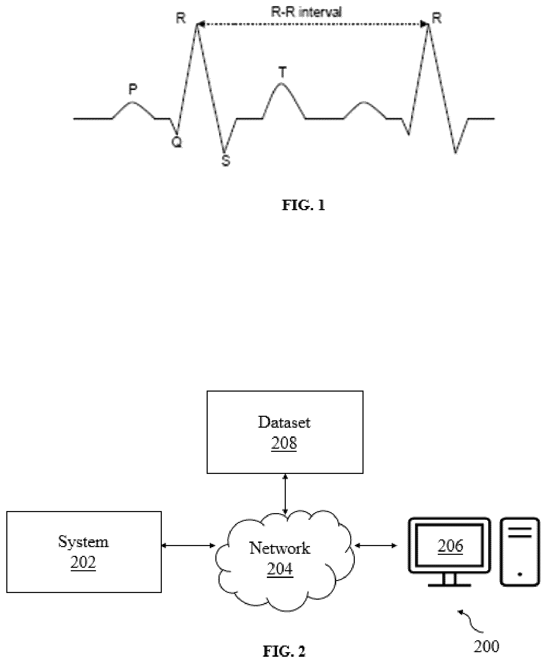

Referring now to the drawings, and more particularly to FIGS. 1 through 7, where similar reference characters denote corresponding features consistently throughout the figures, there are shown preferred embodiments and these embodiments are described in the context of the following exemplary system and/or method.

FIG. 1 illustrates a schematic representation of an electrocardiogram (ECG) waveform, in accordance with some embodiments of the present disclosure.

As seen in FIG. 1, the ECG waveform includes distinct 'P' waves representing atrial depolarization, followed by Q-R-S complexes representing ventricular depolarization, and T waves representing ventricular repolarization. During a normal sinus rhythm, an observed sequence is always P-Q-R-S-T. However, in cases of AF, the morphology of the ECG signal becomes irregular and chaotic. Hence, the AF is best characterized by the irregularity in the R-R intervals. In particular, the irregular conduction of atrial impulses through an atrioventricular node to the ventricles, and the absence of discernible P-waves (a small upward wave preceding the Q-R-S complex), replaced instead by low-amplitude fibrillatory waves reflects presence of the AF.

The morphology of the ECG is explained first as the morphology forms the basis for the functioning of a system explaining decision-making process of deep learning models used for detecting atrial fibrillation (AF) in ECG waves.

FIG. 2 illustrates an exemplary representation of an environment 200 related to at least some example embodiments of the present disclosure. Although the environment 200 is presented in one arrangement, other embodiments may include the parts of the environment 200 (or other parts) arranged otherwise depending on, for example, converting 1D ECG signal into a two-dimensional (2D) ECG image, training a deep learning classifier model based on the 2D ECG image. The environment 200 generally includes a system (BMS) 202, an electronic device 206 (hereinafter also referred as a user device 206) and a dataset 208, each coupled to, and in communication with (and/or with access to) a network 204. It should be noted that one user device is shown for the sake of explanation; there can be more number of user devices.

The network 204 may include, without limitation, a light fidelity (Li-Fi) network, a local area network (LAN), a wide area network (WAN), a metropolitan area network (MAN), a satellite network, the Internet, a fiber optic network, a coaxial cable network, an infrared (IR) network, a radio frequency (RF) network, a virtual network, and/or another suitable public and/or private network capable of supporting communication among two or more of the parts or users illustrated in FIG. 2, or any combination thereof.

Various entities in the environment 200 may connect to the network 204 in accordance with various wired and wireless communication protocols, such as Transmission Control Protocol and Internet Protocol (TCP/IP), User Datagram Protocol (UDP), 2nd Generation (2G), 3rd Generation (3G), 4th Generation (4G), 5th Generation (5G) communication protocols, Long Term Evolution (LTE) communication protocols, or any combination thereof.

Various entities in the environment 200 may connect to the network 104 in accordance with various wired and wireless communication protocols, such as Transmission Control Protocol and Internet Protocol (TCP/IP), User Datagram Protocol (UDP), 2nd Generation (2G), 3rd Generation (3G), 4th Generation (4G), 5th Generation (5G) communication protocols, Long Term Evolution (LTE) communication protocols, or any combination thereof.

The user device 206 is associated with a user (e.g., primary care doctors, gynecologists ) who are not specialized in cardiology but need to take clinical decisions from ECG recordings. Examples of the user device 106 include, but are not limited to, a personal computer (PC), a mobile phone, a tablet device, a Personal Digital Assistant (PDA), a server, a voice activated assistant, a smartphone, and a laptop.

The dataset 208 can a publicly available dataset, such as Physionet 2017 challenge dataset or a customized dataset prepared for experimentation purposes.

The system 202 includes one or more hardware processors and a memory. The system 202 is configured to perform one or more of the operations described herein. The system 202 is configured to receive a raw 1D ECG signal from a data source, such as the data source 208 via the network 204. In an embodiment, the system 202 may receive the 1D ECG signal from a user device, such as the user device 206. The received 1D ECG signal can be captured from a person. The system 202 then converts the 1D ECG signal into a two-dimensional (2D) ECG image using a 1D-2D signal to image conversion algorithm.

Thereafter, the system 202 trains a deep learning classifier model based on the 2D ECG image and a clinical domain knowledge to obtain a trained deep learning classifier model. In an embodiment, the clinical domain knowledge is acquired from one or more domain experts. The trained deep learning classifier model can provide a class label for the 2D ECG image among one or more predefined class labels.

Further, the system 202 generates 2D class activation maps (CAM) by applying the attribution method over the trained deep learning classifier model. The CAM determine a set of regions in the 2D ECG image influencing the deep learning classifier model for predicting the class label from the one or more class labels. The system 202 then converts the 2D ECG image into the 1D ECG signal using a 2D-1D image to signal conversion algorithm which also converts the 2D class activation map into a 1D attention wave.

Additionally, the system 202 calculates a plurality of attention scores from a plurality of R-R intervals present in the 1D ECG signal based on the 1D attention wave. Finally, the system 202 sort the plurality of attention scores to obtain a set of high attention scores corresponding to a set of top R-R intervals into which the trained deep learning classifier model is placing emphasis using a pre-defined threshold value. The set of top R-R intervals provides clinical expert like explanation for the class label prediction performed by the trained deep learning classifier model.

The number and arrangement of systems, devices, and/or networks shown in FIG. 2 are provided as an example. There may be additional systems, devices, and/or networks; fewer systems, devices, and/or networks; different systems, devices, and/or networks; and/or differently arranged systems, devices, and/or networks than those shown in FIG. 2. Furthermore, two or more systems or devices shown in FIG. 2 may be implemented within a single system or device, or a single system or device shown in FIG. 2 may be implemented as multiple, distributed systems or devices. Additionally, or alternatively, a set of systems (e.g., one or more systems) or a set of devices (e.g., one or more devices) of the environment 200 may perform one or more functions described as being performed by another set of systems or another set of devices of the environment 200 (e.g., refer scenarios described above).

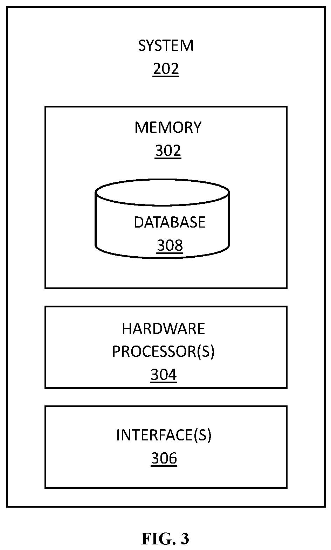

FIG. 3 illustrates an exemplary block diagram of a system 202 for explaining decision-making process of deep learning models used for detecting atrial fibrillation in electrocardiogram (ECG) waves, in accordance with an embodiment of the present disclosure.

In some embodiments, the system 202 is embodied as a cloud-based and/or SaaS-based (software as a service) architecture. In some embodiments, the system 202 may be implemented in a server system. In some embodiments, the system 202 may be implemented in a variety of computing systems, such as laptop computers, notebooks, hand-held devices, workstations, mainframe computers, and the like.

In an embodiment, the system 202 includes one or more processors 304, communication interface device(s) or input/output (I/O) interface(s) 306, and one or more data storage devices or memory 302 operatively coupled to the one or more processors 304. The one or more processors 304 may be one or more software processing modules and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 202 can be implemented in a variety of computing systems, such as laptop computers, notebooks, hand-held devices, workstations, mainframe computers, servers, a network cloud and the like.

The I/O interface device(s) 306 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 302 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment a database 308 can be stored in the memory 302, wherein the database 308 may comprise, but are not limited to pre-defined threshold value, one or more processes and the like. In an embodiment, the memory 302 may store information pertaining to training samples, plug and play language modeling technique, token selection criteria, and the like. The memory 302 further comprises (or may further comprise) information pertaining to input(s)/output(s) of each step performed by the systems and methods of the present disclosure. In other words, input(s) fed at each step and output(s) generated at each step are comprised in the memory 302 and can be utilized in further processing and analysis.

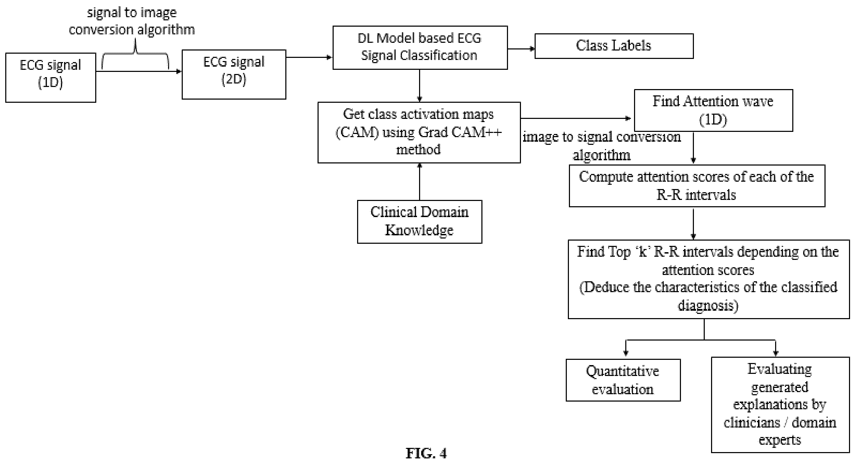

FIG. 4, with reference to FIGS, 1-3, is a schematic flow diagram representation illustrating working of the system 202 of FIGS. 2 and 3, in accordance with an embodiment of the present disclosure.

As seen in FIG. 4, the system 202 receives the 1D ECG signal as input which is converted into a 2D ECG signal using the 1D-2D signal to image conversion algorithm. In an embodiment, the 1D-2D signal to image conversion algorithm used for converting the 1D ECG signal to the 2D ECG signal is already discussed in Indian Patent Application No: 202221050616 filed on 05 September 2022. However, any available 1D-2D signal to image conversion algorithm can be used for the same purpose. Then, the deep learning classifier model performs ECG classification based on the 2D ECG signal to provide a class label for the ECG signal based on one or more predefined class labels. Thereafter, an attribution method, such as Grad CAM++ is applied on the deep learning classifier model to obtain 2D class activation maps.

Further, to generate clinical expert like explanation, the 2D ECG image needs to be converted to the 1D ECG signal using a 2D-1D image to signal conversion algorithm. The 2D-1D image to signal conversion algorithm also converts the 2D CAM into a 1D attention wave while converting the 2D ECG image to the 1D ECG signal. Then, an attention score is computed for each R-R intervals of a plurality of R-R intervals present in the 1D ECG signal based on the 1D attention wave. Thereafter, a set of top 'k' R-R intervals on which the deep learning classifier model is placing more emphasis for determining the class label are selected depending on the attention scores. The set of top 'k' R-R intervals deduce the characteristics of the classified diagnosis, such as for AF (irregular R-R interval, P-waves missing etc.) are deduced similar to the domain knowledge. In particular, the set of top R-R intervals provides clinical expert like explanation for the class label prediction performed by the trained deep learning classifier model.

The system generated clinical expert like explanation are also evaluated by clinicians / domain experts and they also confirmed that the explanations are aligned with the domain knowledge i.e., this is similar to how cardiologists analyze an ECG signal. Hence, these are acceptable to clinical domain experts.

It should be noted that the 'clinical expert like explanation' is basically an explanation on why a particular class label is selected by the deep learning classifier model while performing classification. In particular, it explains a purpose of the classification of the ECG signal and explains the decisions made by the deep learning classifier model.

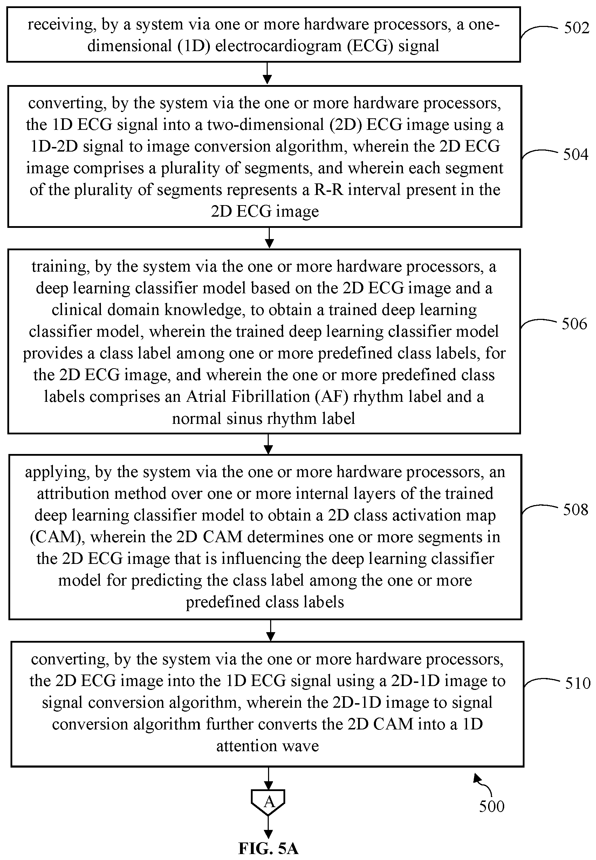

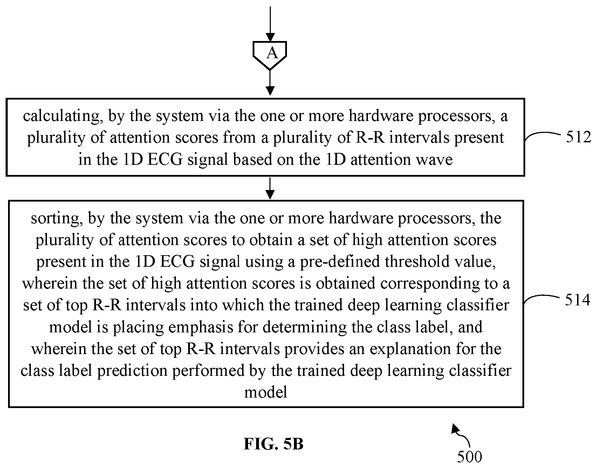

FIG. 5, with reference to FIGS. 1 to 4, illustrates an exemplary flow diagram of a method 500 for explaining decision-making process of deep learning models used for detecting atrial fibrillation in ECG waves, in accordance with an embodiment of the present disclosure. In an embodiment, the system 202 comprises one or more data storage devices or the memory 302 operatively coupled to the one or more hardware processors 304 and is configured to store instructions for execution of steps of the method 500 by the one or more hardware processors 304. The steps of the method 500 of the present disclosure will now be explained with reference to the components of the system 202 as depicted in FIG. 3, and the flow diagram in FIGS. 5A and 5B.

At step 502 of the method of the present disclosure, the one or more hardware processors 304 of the system 202 receive a one-dimensional (1D) electrocardiogram (ECG) signal. The system 202 may receive the 1D ECG signal from the dataset 208 or may receive from the user device 206.

At step 504 of the present disclosure, the hardware processors 304 of the system 202 convert the 1D ECG signal into a two-dimensional (2D) ECG image using a 1D-2D signal to image conversion algorithm. It should be noted that any 1D-2D signal to image conversion algorithm that can generate a 2D representation of the ECG signals can be used for the same purpose. An example 1D-2D signal to image conversion algorithm that can be used for conversion is provided below:

Input: 1D ECG signal $\varepsilon$

1: Detect R-peaks and their corresponding onset and offset on the input signal. Let there be $(N+1)$ R-peaks in $\varepsilon$, indexed at positions $R_n$, for $n = 0, 1, 2, 3,...,N$. Onset and offset corresponding to the R-peak at the position $R_n$ are denoted as $r_n^-$ and $r_n^+$.

2: Define ECG segments $\varepsilon_0 = \varepsilon\left[: r_1^+ + \epsilon\right]$; $\forall n \in \{2, 3,..., N - 1\}$, $\varepsilon_n = \varepsilon\left[: r_{n-1}^- - \epsilon, r_n^+ + \epsilon\right]$; $\varepsilon_n = \varepsilon\left[r_{n-1}^- -:\right]$ for a suitable choice of $\epsilon$. To achieve a uniform width length $\delta\left(\geq max_n\{r_n^+ - r_{n-1}^-\}\right)$, each $\varepsilon_n$ should be adjusted by zero-padding $\mathbb{Z}_n$.

3: Stack the finite sequence of segments $\{\varepsilon_n\}_{n=1}^N$.

Output: 2D matrix representation of $\varepsilon = \varepsilon_{2D}, \{\mathbb{Z}_n\}_{n=1}^{N}, \epsilon$.

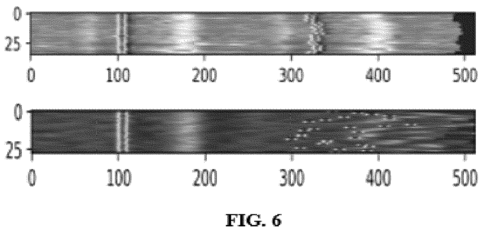

The 2D ECG image includes a plurality of segments, and each segment of the plurality of segments represents a R-R interval present in the 2D ECG image. An example representation of the 2D ECG image is shown with respect to FIG. 6.

At step 506 of the present disclosure, the hardware processors 304 of the system 202 train a deep learning classifier model based on the 2D ECG image and a clinical domain knowledge, to obtain a trained deep learning classifier model. In an embodiment, the clinical domain knowledge is disease specific domain knowledge i.e., it is related with atrial fibrillation (AF) and is acquired from disease specific domain experts, such as cardiologists.

The deep learning classifier model, once trained, provides a class label for the 2D ECG image among one or more predefined class labels. In an embodiment, the one or more predefined class labels comprises an Atrial Fibrillation (AF) rhythm label and a normal sinus rhythm label.

At step 508 of the present disclosure, the hardware processors 304 of the system 202 applies an attribution method over one or more internal layers of the trained deep learning classifier model to obtain a 2D class activation map (CAM). It should be noted that as the ECG is represented in image format i.e. the 2D ECG image is available, any computer vision based attribution method, such as Grad CAM++ can be applied to obtain 2D CAM. In an embodiment, the 2D CAM determines one or more segments in the 2D ECG image that are influencing the deep learning classifier model for predicting the class label among the one or more predefined class labels. In particular, the 2D CAM helps in identifying one or more important regions of an input image influencing the classifier network's prediction of a specific class. However, the CAM do not provide clear insights for clinical domain experts. For instance, these maps does not let one to deduce whether the R-R interval serves as a crucial feature utilized by the deep learning classifier model. So, to mitigate this concern, the system 202 performs back transformation from 2D ECG image into the 1D ECG signal at step 510.

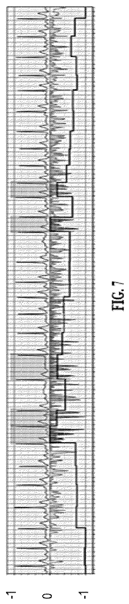

At step 510 of the present disclosure, the hardware processors 304 of the system 202 convert the 2D ECG image into the 1D ECG signal using a 2D-1D image to signal conversion algorithm. An example representation of the 1D ECG signal obtained from the 2D ECG image is shown with respect to FIG. 7.

In an embodiment, for converting the 2D ECG image into the 1D ECG signal using the 2D-1D image to signal conversion algorithm, the system 202 first scans each segment of the plurality of segments present in the 2D ECG image. Then, the system 202 discards each segment that is zero-padded amongst the plurality of segments to obtain a subset of image segments. In particular, for segments which were zero-padded to create the fixed size 2D ECG image, their zero padded portion is discarded while creating the 1D signal. Finally, the system 202 concatenates each segment of the subset of image segments from a first segment to a last segment of the subset of image segments to generate the 1D ECG signal. The 2D-1D image to signal conversion algorithm used above is defined below:

Input: 2D ECG image representation $\varepsilon_{2D}, \{\mathbb{Z}_n\}_{n=1}^{N}, \epsilon$

1: for each row $r_n$ in $\varepsilon_{2D}$ do

2: discard elements in $r_n$ indexed by $\mathbb{Z}_n$ (elements which are zero-padded) to get $\varepsilon_n$

3: Define $T_1 = \varepsilon_1 [: -\epsilon]; \forall n \in \{2, 3, \ldots, N - 1\}, T_n = \varepsilon_n [\epsilon : -\epsilon]; T_n = \varepsilon_n [\epsilon :]$

4: $\varepsilon = []$

5: for $1 \leq n \leq N$ do

6: $\varepsilon = \varepsilon + T_n$, where '+' represents 'append' operation.

Output: 1D ECG Signal $\varepsilon$.

In an embodiment, the 2D-1D image to signal conversion algorithm also converts the 2D CAM into a 1D attention wave. In at least one example embodiment, for converting the 2D CAM into a 1D attention wave using the 2D-1D image to signal conversion algorithm, the system 202 first scans each each CAM segment of a plurality of CAM segments present in the 2D CAM. Then, the system 202 discards each CAM segment that is zero-padded amongst the plurality of CAM segments to obtain a subset of CAM segments. Finally, the system 202 concatenates each CAM segment of the subset of CAM segments from a first CAM segment to a last CAM segment of the subset of CAM segments to generate the 1D attention wave.

At step 512 of the present disclosure, the hardware processors 304 of the system 202 calculate a plurality of attention scores from a plurality of R-R intervals present in the 1D ECG signal based on the 1D attention wave. The above step is better understood by way of following description.

From clinical domain knowledge, the characteristics and morphology of an ECG signal like R-R intervals, P waves, etc. is already known. So, the 1D attention wave is used to compute the attention scores of such morphological elements.

For calculating the plurality of attention scores, the system 202 first scan each R-R interval of the plurality of R-R intervals present in the 1D ECG signal to obtain a length corresponding to each R-R interval. Then, the system 202 computes an area corresponding to the 1D attention wave under each R-R interval using the Trapezoidal Rule. Thereafter, the system 202 normalizes the area for each R-R interval by dividing the area with the length of each R-R interval to obtain an attention score. For instance, for an R-R interval which starts at time step 'a' and ends at time step 'b', the beat level attention ($B_{AT}$) can be defined as:

$$B_{AT} = \frac{\int_a^b A(r)dr}{(b-a)} \approx \frac{\sum_{i=1}^N \frac{A(r_{i-1}) + A(r_i)}{2} \Delta r_i}{(b-a)}$$

Where, $\{r_i\}$ is a partition of $[a, b]$, $\Delta r_i = (r_i - r_{i-1})$.

The attention scores obtained corresponding to the plurality of R-R intervals are referred as the plurality of attention scores.

At step 514 of the present disclosure, the hardware processors 304 of the system 202 sort the plurality of attention scores to obtain a set of high attention scores present in the 1D ECG signal using a pre-defined threshold value. The set of high attention scores is obtained corresponding to a set of top R-R intervals into which the trained deep learning classifier model is placing emphasis for determining the class label. The set of top R-R intervals provides an explanation (i.e., the clinical expert like explanation) for the class label prediction performed by the trained deep learning classifier model. In particular, by examining the top R-R intervals, one can easily gain insight into whether the deep learning classifier model is prioritizing irregularities in the R-R intervals and the absence of P waves or not while detecting AF.

The top R-R intervals are then marked on the ECG trace to get the 1D ECG signal plots with the generated clinical expert like explanations. In an embodiment, the ECG signal plots with the generated clinical expert like explanations are displayed on the user device 206.

FIG. 6 illustrates an example representation of a 2D ECG image obtained from a 1D ECG signal, in accordance with an embodiment of the present disclosure.

As seen in FIG.6, an x-axis is fixed to 512 length and a y-axis represents a number of rows i.e. a number of segments (number of R-R intervals) present in an ECG signal.

FIG. 7 is an example representation of the one-dimensional ECG signal obtained from the two-dimensional ECG image, in accordance with an embodiment of the present disclosure.

As seen in FIG.7, top R-R intervals are highlighted showing the important regions (attention wave) where the deep learning classifier model is focusing for performing the class label prediction.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

As discussed earlier, existing deep learning based methods for classifying ECG signal lacks the explanation of why this decision has been made, hence it is not well accepted by the clinical domain experts. To overcome the disadvantages, embodiments of the present disclosure provide the method and the system for explaining decision-making process of deep learning models used for detecting atrial fibrillation in electrocardiogram (ECG) waves. More specifically, the system converts the class activation maps into a 1D attention wave which along with the clinical domain knowledge helps in investigating specific region of interests (ROIs) in the input ECG. The ROIs further helps in gaining insight into whether the deep learning classifier model is prioritizing irregularities in the R-R intervals and the absence of P waves to come up with classification or not, thus providing clinical expert like explanations for the classification decision while ensuring increased accuracy of the AF detection. Further, the system approach is closely aligned with how cardiologists analyze an ECG signal, thereby making the diagnosis more acceptable for clinical domain experts.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

**1.** A processor implemented method (500), comprising:

receiving (502), by a system via one or more hardware processors, a one-dimensional (1D) electrocardiogram (ECG) signal;
converting (504), by the system via the one or more hardware processors, the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image;
training (506), by the system via the one or more hardware processors, a deep learning classifier model based on the 2D ECG image and a clinical domain knowledge to obtain a trained deep learning classifier model, wherein the trained deep learning classifier model provides a class label among one or more predefined class labels, for the 2D ECG image, wherein the one or more predefined class labels comprises an Atrial Fibrillation (AF) rhythm label and a normal sinus rhythm label;
applying (508), by the system via the one or more hardware processors, an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels;

converting (510), by the system via the one or more hardware processors, the 2D ECG image into the 1D ECG signal using a 2D-1D image to signal conversion algorithm, wherein the 2D-1D image to signal conversion algorithm further converts the 2D CAM into a 1D attention wave;

calculating (512), by the system via the one or more hardware processors, a plurality of attention scores from a plurality of R-R intervals present in the 1D ECG signal based on the 1D attention wave; and

sorting (514), by the system via the one or more hardware processors, the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

2. The processor implemented method as claimed in claim 1, wherein the 2D-1D image to signal conversion algorithm converts the 2D ECG image into the 1D ECG signal by performing:

scanning each segment of the plurality of segments present in the 2D ECG image;
discarding each segment amongst the plurality of segments that is zero-padded to obtain a subset of image segments; and
concatenating each segment of the subset of image segments from a first segment to a last segment of the subset of image segments to generate the 1D ECG signal.

3. The processor implemented method as claimed in claim 1, wherein the 2D-1D image to signal conversion algorithm converts the 2D CAM into the 1D attention wave by performing:

scanning each each CAM segment of a plurality of CAM segments present in the 2D CAM;
discarding each CAM segment amongst the plurality of CAM segments that is zero-padded to obtain a subset of CAM segments; and
concatenating each CAM segment of the subset of CAM segments from a first CAM segment to a last CAM segment of the subset of CAM segments to generate the 1D attention wave.

4. The processor implemented method as claimed in claim 1, wherein the plurality of attention scores are calculated by performing:

scanning, by the system via the one or more hardware processors, each R-R interval of the plurality of R-R intervals present in the 1D ECG signal to obtain a length corresponding to each R-R interval;
computing, by the system via the one or more hardware processors, an area corresponding to the 1D attention wave under each R-R interval using a Trapezoidal Rule; and
normalizing, by the system via the one or more hardware processors, the area for each R-R interval by dividing the area with the length of each R-R interval to obtain an attention score, wherein attention scores obtained corresponding to the plurality of R-R intervals form the plurality of attention scores.

5. A system (202), comprising:

a memory (302) storing instructions;
one or more communication interfaces (306); and
one or more hardware processors (304) coupled to the memory (302) via the one or more communication interfaces (306), wherein the one or more hardware processors (304) are configured by the instructions to:

receive a one-dimensional (1D) electrocardiogram (ECG) signal;
convert the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image;
train a deep learning classifier model based on the 2D ECG image and a clinical domain knowledge, to obtain a trained deep learning classifier model, wherein the trained deep learning classifier model provides a class label among one or more predefined class labels, for the 2D ECG image, wherein the one or more predefined class labels comprises an Atrial Fibrillation (AF) rhythm label and a normal sinus rhythm label;
apply an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels;

convert the 2D ECG image into the 1D ECG signal using a 2D-1D image to signal conversion algorithm, wherein the 2D-1D image to signal conversion algorithm further converts the 2D CAM into a 1D attention wave;

calculate a plurality of attention scores from a plurality of R-R intervals present in the 1D ECG signal based on the 1D attention wave; and

sort the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

6. The system (202) as claimed in claim 5, wherein for converting the 2D ECG image into the 1D ECG signal using the 2D-1D image to signal conversion algorithm, the one or more hardware processors (304) are configured by the instructions to:

scan each segment of the plurality of segments present in the 2D ECG image;

discard each segment amongst the plurality of segments that is zero-padded to obtain a subset of image segments; and

concatenate each segment of the subset of image segments from a first segment to a last segment of the subset of image segments to generate the 1D ECG signal.

7. The system (202) as claimed in claim 5, wherein for converting the 2D CAM into the 1D attention wave using the 2D-1D image to signal conversion algorithm, the one or more hardware processors (304) are configured by the instructions to:

scan each each CAM segment of a plurality of CAM segments present in the 2D CAM;

discard each CAM segment amongst the plurality of CAM segments that is zero-padded to obtain a subset of CAM segments; and

concatenate each CAM segment of the subset of CAM segments from a first CAM segment to a last CAM segment of the subset of CAM segments to generate the 1D attention wave.

8. The system (202) as claimed in claim 5, wherein for calculating the plurality of attention scores, the one or more hardware processors (304) are configured by the instructions to:

scan each R-R interval of the plurality of R-R intervals present in the 1D ECG signal to obtain a length corresponding to each R-R interval;

compute an area corresponding to the 1D attention wave under each R-R interval using a Trapezoidal Rule; and

normalize the area for each R-R interval by dividing the area with length of each R-R interval to obtain an attention score, wherein attention scores obtained corresponding to the plurality of R-R intervals form the plurality of attention scores.

9. One or more non-transitory machine-readable information storage mediums comprising one or more instructions which when executed by one or more hardware processors cause:

receiving, by a system, a one-dimensional electrocardiogram (ECG) signal;

converting, by the system, the 1D ECG signal into a two-dimensional ECG image using a 1D-2D signal to image conversion algorithm, wherein the 2D ECG image comprises a plurality of segments, and wherein each segment of the plurality of segments represents a R-R interval present in the 2D ECG image;

training, by the system a deep learning classifier model based on the 2D ECG image and a clinical domain knowledge to obtain a trained deep learning classifier model, wherein the trained deep learning classifier model provides a class label among one or more predefined class labels, for the 2D ECG image, wherein the one or more predefined class labels comprises an Atrial Fibrillation (AF) rhythm label and a normal sinus rhythm label;

applying, by the system, an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels;

converting, by the system, the 2D ECG image into the 1D ECG signal using a 2D-1D image to signal conversion algorithm, wherein the 2D-1D image to signal conversion algorithm further converts the 2D CAM into a 1D attention wave;

calculating, by the system, a plurality of attention scores from a plurality of R-R intervals present in the 1D ECG signal based on the 1D attention wave; and

sorting, by the system, the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model.

10. one or more non-transitory machine-readable information storage mediums of claim 9, wherein the 2D-1D image to signal conversion algorithm converts the 2D ECG image into the 1D ECG signal by performing:

scanning each segment of the plurality of segments present in the 2D ECG image;

discarding each segment amongst the plurality of segments that is zero-padded to obtain a subset of image segments; and

concatenating each segment of the subset of image segments from a first segment to a last segment of the subset of image segments to generate the 1D ECG signal.

11. The one or more non-transitory machine-readable information storage mediums of claim 9, wherein the 2D-1D image to signal conversion algorithm converts the 2D CAM into the 1D attention wave by performing:

scanning each each CAM segment of a plurality of CAM segments present in the 2D CAM;

discarding each CAM segment amongst the plurality of CAM segments that is zero-padded to obtain a subset of CAM segments; and

concatenating each CAM segment of the subset of CAM segments from a first CAM segment to a last CAM segment of the subset of CAM segments to generate the 1D attention wave.

12. The one or more non-transitory machine-readable information storage mediums of claim 9, wherein the plurality of attention scores are calculated by performing:

scanning, by the system, each R-R interval of the plurality of R-R intervals present in the 1D ECG signal to obtain a length corresponding to each R-R interval;

computing, by the system, an area corresponding to the 1D attention wave under each R-R interval using a Trapezoidal Rule; and

normalizing, by the system, the area for each R-R interval by dividing the area with the length of each R-R interval to obtain an attention score, wherein attention scores obtained corresponding to the plurality of R-R intervals form the plurality of attention scores.

**FIG. 1**

**FIG. 2**

SYSTEM
202

MEMORY
302

DATABASE
308

HARDWARE
PROCESSOR(S)
304

INTERFACE(S)
306

**FIG. 3**

signal to image
conversion algorithm

ECG signal
(1D)

ECG signal
(2D)

DL Model based ECG
Signal Classification

Class Labels

Get class activation maps
(CAM) using Grad CAM++
method

Find Attention wave
(1D)

image to signal conversion
algorithm

Clinical Domain
Knowledge

Compute attention scores of each of the
R-R intervals

Find Top 'k' R-R intervals depending on the
attention scores
(Deduce the characteristics of the classified
diagnosis)

Quantitative
evaluation

Evaluating
generated
explanations by
clinicians / domain
experts

FIG. 4

receiving, by a system via one or more hardware processors, a one-dimensional (1D) electrocardiogram (ECG) signal ⌐ 502

↓

converting, by the system via the one or more hardware processors, the 1D ECG signal into a two-dimensional (2D) ECG image using a 1D-2D signal to image conversion algorithm, wherein the 2D ECG image comprises a plurality of segments, and wherein each segment of the plurality of segments represents a R-R interval present in the 2D ECG image ⌐ 504

↓

training, by the system via the one or more hardware processors, a deep learning classifier model based on the 2D ECG image and a clinical domain knowledge, to obtain a trained deep learning classifier model, wherein the trained deep learning classifier model provides a class label among one or more predefined class labels, for the 2D ECG image, and wherein the one or more predefined class labels comprises an Atrial Fibrillation (AF) rhythm label and a normal sinus rhythm label ⌐ 506

↓

applying, by the system via the one or more hardware processors, an attribution method over one or more internal layers of the trained deep learning classifier model to obtain a 2D class activation map (CAM), wherein the 2D CAM determines one or more segments in the 2D ECG image that is influencing the deep learning classifier model for predicting the class label among the one or more predefined class labels ⌐ 508

↓

converting, by the system via the one or more hardware processors, the 2D ECG image into the 1D ECG signal using a 2D-1D image to signal conversion algorithm, wherein the 2D-1D image to signal conversion algorithm further converts the 2D CAM into a 1D attention wave ⌐ 510

↓

A

500

**FIG. 5A**

A

calculating, by the system via the one or more hardware processors, a plurality of attention scores from a plurality of R-R intervals present in the 1D ECG signal based on the 1D attention wave ⌐ 512

sorting, by the system via the one or more hardware processors, the plurality of attention scores to obtain a set of high attention scores present in the 1D ECG signal using a pre-defined threshold value, wherein the set of high attention scores is obtained corresponding to a set of top R-R intervals into which the trained deep learning classifier model is placing emphasis for determining the class label, and wherein the set of top R-R intervals provides an explanation for the class label prediction performed by the trained deep learning classifier model ⌐ 514

**FIG. 5B**

500

FIG. 6

FIG. 7

# EUROPEAN SEARCH REPORT

**Application Number**

EP 25 18 4341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOUSAVI SAJAD ET AL: "HAN-ECG: An interpretable atrial fibrillation detection model using hierarchical attention networks", COMPUTERS IN BIOLOGY AND MEDICINE, NEW YORK, NY, US, vol. 127, 15 October 2020 (2020-10-15), XP086417278, ISSN: 0010-4825, DOI: 10.1016/J.COMPBIOMED.2020.104057 [retrieved on 2020-10-15] * page 2, column 1, line 35 - line 59; figures 5,6 * * page 3, column 1, line 8 - page 5, column 1, line 9 * * page 5, column 2, line 51 - line 57 * | 1-12 | INV. A61B5/361 A61B5/00 |
| A,D | ----- EP 4 331 488 A1 (TATA CONSULTANCY SERVICES LTD [IN]) 6 March 2024 (2024-03-06) * paragraph [0010] - paragraph [0035]; figure 2 * | 1-12 | |
| A | ----- LIU JIKUI ET AL: "Myocardial Infarction Detection and Localization with Electrocardiogram Based on Convolutional Neural Network", CHINESE JOURNAL OF ELECTRONICS, TECHNOLOGY EXCHANGE LTD., HONG KONG, HK, vol. 30, no. 5, 1 September 2021 (2021-09-01), pages 833-842, XP006113429, ISSN: 1022-4653, DOI: 10.1049/CJE.2021.06.005 * page 834, column 2, line 37 - page 841, column 2, line 3; figure 2 * ----- -/-- | 1-12 | **TECHNICAL FIELDS SEARCHED (IPC)** A61B |

The present search report has been drawn up for all claims

| Place of search | Date of completion of the search | Examiner |
|---|---|---|
| The Hague | 25 August 2025 | Bourquin, Yannyk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

# EUROPEAN SEARCH REPORT

Application Number

EP 25 18 4341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SONG MIN-SEO ET AL: "Comparative study of time-frequency transformation methods for ECG signal classification", FRONTIERS IN SIGNAL PROCESSING, vol. 4, 29 January 2024 (2024-01-29), XP093306212, ISSN: 2673-8198, DOI: 10.3389/frsip.2024.1322334 * page 2, column 2, line 40 - page 11, column 2, line 15 * ----- | 1-12 | |
| A | CN 115 363 599 A (UNIV SHANDONG) 22 November 2022 (2022-11-22) * paragraph [0006] - paragraph [0153] * ----- | 1-12 | |

TECHNICAL FIELDS SEARCHED (IPC)

The present search report has been drawn up for all claims

| Place of search | Date of completion of the search | Examiner |
|---|---|---|
| The Hague | 25 August 2025 | Bourquin, Yannyk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 4341

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2025

| Patent document cited in search report | | Publication date | Patent family member(s) | | Publication date |
|---|---|---|---|---|---|
| EP 4331488 | A1 | 06-03-2024 | EP 4331488 A1 | | 06-03-2024 |
| | | | US 2024079140 A1 | | 07-03-2024 |
| CN 115363599 | A | 22-11-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421052050

- IN 202221050616